# FASCICULE DE BREVET EUROPEEN

(11) **EP 3 629 994 B1**
(45) Date de publication et mention de la délivrance du brevet: **18.01.2023**
(21) Numéro de dépôt: 18729625.6
(22) Date de dépôt: 31.05.2018
(51) Int. Cl.: A61C 7/08, A61C 7/36, A61F 5/56, A61F 5/50

(54) **APPAREIL ORTHODONTIQUE ET PROCEDE DE FABRICATION D'UN TEL APPAREIL**
KIEFERORTHOPÄDISCHE VORRICHTUNG UND VERFAHREN ZUR HERSTELLUNG EINER SOLCHEN VORRICHTUNG
ORTHODONTIC APPLIANCE AND METHOD FOR MANUFACTURING SUCH AN APPLIANCE

(30) Priorité: 01.06.2017 FR 1754874
(43) Date de publication de la demande: 08.04.2020
(73) Titulaire: Nutislab, 64200 Biarritz (FR)
(72) Inventeur: CHAZALON, Laurent, 64100 Bayonne (FR); BRETON, Julien, 64310 Sare (FR); PECHEVIS, Véronique, 64210 Bidart (FR)
(74) Mandataire: Decorchemont, Audrey Véronique Christèle
(86) Numéro de dépôt international: PCT/EP2018/064385
(87) Numéro de publication internationale: WO 2018/220145

(56) Documents cités:
- WO-A1-92/05752
- WO-A1-92/09249
- KR-A- 20090 090 969
- US-A- 4 997 182
- US-A1- 2007 079 833

## Description

La présente invention concerne un appareil orthodontique.

L'invention concerne également un procédé de fabrication d'un tel appareil orthodontique.

### ARRIERE PLAN DE L'INVENTION

Chez l'être humain, l'organe buccal comporte le vestibule et la cavité buccale, séparés l'un l'autre par les arcs dentaires. La mâchoire supérieure, parfois appelée maxillaire, porte l'arc dentaire supérieur et la mâchoire inférieure, parfois appelée mandibule, porte l'arc dentaire inférieur. Chez un patient ayant une occlusion dentaire normale, les arcs dentaires inférieur et supérieur sont sensiblement de mêmes dimensions de sorte que lorsque les mâchoires supérieure et inférieure sont fermées, l'arc dentaire supérieur repose sur l'arc dentaire inférieur.

En revanche, chez un patient présentant une malocclusion, il n'est pas rare qu'un mauvais placement de la langue du patient en soit la principale cause.

En effet, la langue doit normalement être positionnée dans la cavité buccale de sorte que sa pointe touche le palais lorsque la langue est au repos mais également lors d'une déglutition. Cet apprentissage du bon placement de la langue est normalement inné et s'effectue sur les premières années de vie d'un enfant.

Toutefois, cet apprentissage n'est pas toujours effectué, ou alors de manière incorrecte, et certains enfants conservent ainsi un positionnement de la langue tel que l'enfant appuie sur l'arc dentaire supérieur et/ou l'arc dentaire inférieur au repos ou lors d'une déglutition. Ceci finit par entraîner une déformation des arcs dentaires et donc une malocclusion correspondante.

Afin de résorber ce problème, il est possible de travailler avec un orthophoniste qui va apprendre à l'enfant comment positionner correctement sa langue dans la cavité buccale.

Il est également possible d'avoir recours à des appareils orthodontiques conçus spécifiquement pour la rééducation linguale.

Néanmoins ces appareils sont généralement lourds, volumineux et donc peu confortables à porter lors des séances de rééducation.

Il est connu des documents WO-A-92/09249 et US-A-2007/079833 un appareil fixé au palais du patient grâce à un ancrage dentaire.

### OBJET DE L'INVENTION

Un but de l'invention est de proposer un appareil orthodontique de volume réduit pour un patient.

Un but de l'invention est également de proposer un procédé de fabrication d'un tel appareil orthodontique.

### DEFINITION GENERALE DE L'INVENTION

En vue de la réalisation de ce but, on propose un appareil orthodontique selon la revendication 1.

Dans cette position l'appareil orthodontique s'étend derrière au moins l'arc dentaire supérieur. L'appareil s'avère ainsi simple à positionner et ne gêne pas la fermeture des mâchoires du patient, ou très peu, ce qui rend l'appareil très ergonomique pour le patient.

En outre, l'appareil ne s'étendant en regard de la face vestibulaire de l'arc dentaire supérieur, il s'avère peu visible de l'extérieur ce qui le rend plus esthétique.

Il peut ainsi être envisagé que le patient porte l'appareil au quotidien permettant une rééducation plus rapide et plus efficace.

Selon un mode de réalisation particulier, la face interne du support est conformée pour reproduire la forme du palais.

Selon un mode de réalisation particulier, la bordure périphérique est lisse.

Selon un mode de réalisation particulier, la bordure est conformée globalement en U.

Selon un mode de réalisation particulier, la bordure présente une hauteur supérieure à une hauteur de l'arc dentaire supérieur afin de dépasser du plan d'occlusion.

Selon un mode de réalisation particulier, la bordure s'étend au moins sur la hauteur combinée de l'arc dentaire supérieur et de l'arc dentaire inférieur.

Selon un mode de réalisation particulier, la bordure s'étend entre 1 millimètre et 3 millimètres en retrait d'une face palatine de l'arc dentaire supérieur.

Selon un mode de réalisation particulier, l'appareil comporte une zone d'accueil de la langue du patient comprenant un orifice.

Selon un mode de réalisation particulier, l'appareil comporte une zone d'accueil de la langue du patient, la zone d'accueil comportant une zone en relief.

Selon un mode de réalisation particulier, la zone d'accueil est conformée en un dessin.

L'invention concerne également un procédé de fabrication d'un appareil orthodontique comprenant les étapes de :
- obtenir une empreinte en trois dimensions d'au moins une partie de la cavité buccale du patient,
- créer dans ladite empreinte au moins un plan au niveau des faces occlusales des deux arcs dentaires de ladite empreinte pour modéliser au moins une partie du palais du patient,
- créer une modélisation en trois dimensions de l'appareil orthodontique,
- créer l'appareil orthodontique à partir de ladite modélisation.

Optionnellement, le plan est créé afin qu'il croise le plus de dents possibles.

Optionnellement, le plan est créé afin qu'il croise en priorité et au maximum les dents les plus rentrées dans la cavité buccale.

Optionnellement, on créé dans l'empreinte au moins deux autres plans, l'un associé à l'arc dentaire supérieur et l'autre à l'arc dentaire inférieur pour modéliser au moins une partie du palais du patient.

Optionnellement, on définit une courbe passant par au moins cinq points du plan.

Optionnellement, on prend également en compte la papille palatine et les bords latéraux de la mandibule pour la modélisation en trois dimensions de l'appareil orthodontique.

D'autres caractéristiques et avantages de l'invention apparaitront plus clairement à la lumière de la description qui va suivre et des dessins annexés, concernant des modes particuliers de réalisation de l'invention.

### BREVE DESCRIPTION DES DESSINS

Il sera fait référence aux figures des dessins annexés, où :
- la figure 1 est une vue en perspective d'un appareil orthodontique selon un premier mode de réalisation de l'invention,
- la figure 2 est une vue en perspective de l'appareil orthodontique illustré à la figure 1 selon une deuxième orientation,
- la figure 3 est une vue en perspective de l'appareil orthodontique illustré à la figure 1 selon une troisième orientation,
- les figures 4 à 6 illustrent différentes étapes d'un procédé de fabrication particulier de l'appareil illustré à la figure 1,
- la figure 7 est une vue en perspective d'un appareil orthodontique selon un deuxième mode de réalisation de l'invention.

### DESCRIPTION DETAILLEE DE MODES DE REALISATION PARTICULIERS DE L'INVENTION

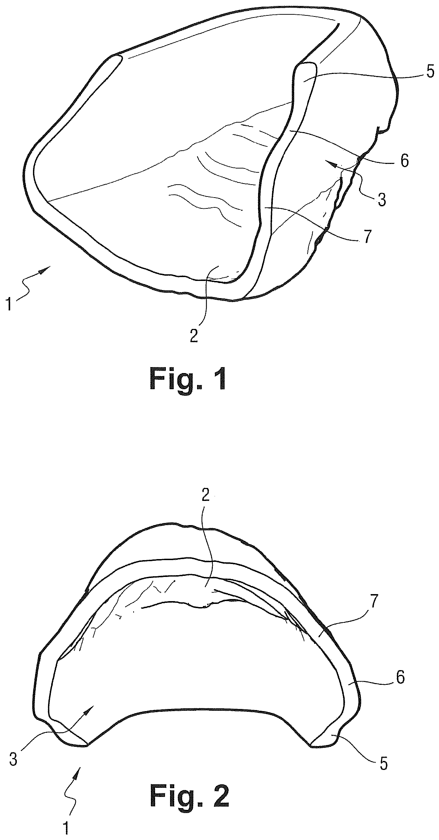

En référence aux figures 1 à 3, l'appareil orthodontique selon un premier mode de réalisation de l'invention, généralement désigné en 1, est ici utilisé pour la rééducation linguale d'un patient. Cette application n'est bien entendu pas limitative et on pourra utiliser l'appareil 1 pour une autre application comme par exemple pour agrandir les arcs dentaires.

L'appareil 1 comprend un support 2 comprenant une face principale externe et une face principale interne. L'appareil 1 est ainsi conformé de sorte que lorsque la face principale externe repose contre le palais du patient, elle est ajustée à la forme du palais du patient.

De façon particulière, la face principale externe épouse la forme du palais. On note que le support 2 est ici de dimensions réduites vis-à-vis de celles du palais du patient : néanmoins sur la partie du palais qu'elle recouvre, la face principale externe épouse bien la forme du palais. La face principale externe est ainsi conformée en empreinte dudit palais.

De manière particulière, c'est ici tout le support 2 qui épouse la forme du palais. De la sorte, la face principale externe épouse la forme du palais lorsque le support 2 y est déposé ce qui permet de coller, par effet « ventouse », l'appareil orthodontique au palais. Par ailleurs, la face principale interne du support 2 simule le palais du patient. Ceci permet au patient de pouvoir passer plus facilement d'exercices effectués avec l'appareil 1 aux exercices effectués sans l'appareil 1.

Par ailleurs, l'appareil 1 comporte une bordure 3 périphérique du support 2.

L'appareil 1 est ainsi conformé de sorte à ne pas s'étendre au niveau des faces vestibulaires des arcs dentaires supérieurs et inférieurs (sauf cas extrême de malocclusion non souhaité) mais seulement à l'intérieur de la cavité buccale.

La bordure 3 est par ailleurs lisse tant sur sa face principale interne que sur sa face principale externe. La bordure 3 ne présente donc pas un aspect texturé comme le support 2 qui vise ici à simuler le palais du patient.

De la sorte, la face principale interne de l'appareil 1 ne présente pas d'arrête ce qui évite un risque de blessure du patient.

La bordure 3 est ici ouverte. Ladite bordure 3 ne borde donc qu'une partie du support 2. La bordure 3 est typiquement conformée de sorte à simuler un arc dentaire.

Ainsi lorsque l'appareil 1 est en place dans la cavité buccale du patient, le support 2 repose contre le palais et la bordure 3 s'étend sensiblement sur tout le long de l'arc dentaire supérieur, en regard de la face palatine de l'arc dentaire supérieur. En revanche, la partie du support 2 la plus proche du fond de la cavité buccale, vers la luette, n'est pas prolongée par la bordure 3.

La bordure 3 est sensiblement conformée en U pour suivre l'arc dentaire.

De façon particulière, la bordure 3 présente une hauteur supérieure à la hauteur de l'arc dentaire supérieur afin de dépasser au moins le plan d'occlusion, lorsque l'appareil 1 est en place dans la cavité buccale et que le patient ferme ses mâchoires. De préférence, la bordure 3 s'étend sur sensiblement la hauteur combinée de l'arc dentaire supérieur et de l'arc dentaire inférieur lorsque l'appareil 1 est en place dans la cavité buccale et que le patient ferme ses mâchoires.

Ceci permet de mieux protéger les dents du patient lors des exercices de rééducation linguale. En particulier, on s'assure ainsi que la langue du patient demeure à l'intérieur du volume défini par l'appareil 1 ce qui évite que la langue ne vienne au contact de l'une des dents.

La bordure 3 comprend :
- une première portion 5 formant l'extrémité libre de la bordure,
- une deuxième portion 6 s'étendant dans le prolongement de la première portion 5, et
- une troisième portion 7 s'étendant dans le prolongement de la deuxième portion 6 et raccordant la bordure 3 au support 2.

Lorsque l'appareil 1 est agencé dans la cavité buccale, la première portion 5 s'étend au niveau de l'arc dentaire inférieur et la deuxième portion 6 au niveau de l'arc dentaire supérieur. Typiquement, la première portion 5 s'étend sensiblement du collet de l'arc dentaire inférieur jusqu'à sensiblement le plan d'occlusion, la deuxième portion 6 s'étend sensiblement du plan d'occlusion jusqu'à sensiblement le collet de l'arc dentaire supérieur et la troisième portion 7 s'étend sensiblement depuis le collet de l'arc dentaire supérieur jusqu'aux bords du palais.

L'appareil 1 est conformé de sorte que lorsque l'appareil 1 est en position dans la cavité buccale, la bordure 3 ne soit pas en contact avec les dents du patient.

Ceci permet d'assurer que l'appareil 1 puisse être utilisé même si le patient porte déjà des bagues dentaires ou tout autre dispositif d'orthodontie.

Typiquement, l'appareil 1 est conformé de sorte qu'en service la bordure 3 s'étende entre 1 millimètre et 3 millimètres, et de préférence entre 1.5 et 2.5 millimètres, en retrait de la face palatine de l'arc dentaire supérieur et de la face linguale de l'arc dentaire inférieur.

De préférence, et comme on le verra par la suite, la bordure est définie au moins en partie en fonction de la forme de la cavité buccale du patient. En particulier, la troisième portion 7 est conformée pour être ajustée à la forme du palais du patient.

Ceci permet de favoriser l'attache de l'appareil 1 au palais par simple adhésion.

L'appareil 1 est bien entendu dans un matériau adapté à l'anatomie humaine. L'appareil 1 est typiquement en résine biocompatible.

De façon particulière, l'épaisseur de l'appareil 1 est comprise entre 1 et 3 millimètres, et de préférence entre 1,2 et 2,5 millimètres. La première portion 5 présente une épaisseur plus importante que le reste de l'appareil 1 (soit ici que les deux autres portions 6, 7 et le support 2).

Préférentiellement on choisit une épaisseur de l'appareil 1, à l'exception de la première portion, comprise entre sensiblement 1.2 et 2 millimètres et une épaisseur de la première portion 5 comprise entre sensiblement 1.8 et 2.2 millimètres. Typiquement l'épaisseur de l'appareil 1, à l'exception de la première portion 5, est de sensiblement 1.5 millimètres.

Un procédé de fabrication d'un tel appareil 1 va être à présent décrit.

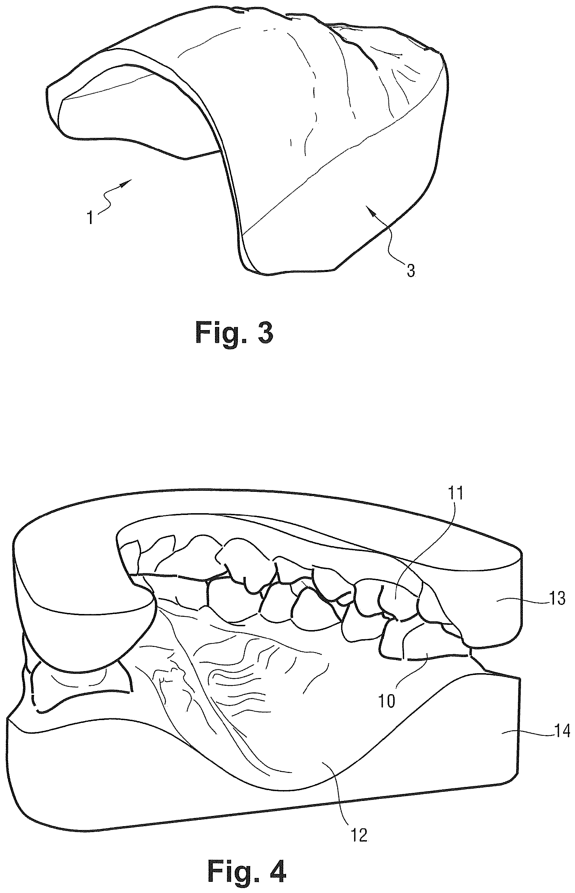

En référence à la figure 4, et selon une première étape, on obtient une empreinte numérique en trois dimensions d'au moins une partie de la cavité buccale d'un patient lorsque ses deux mâchoires sont fermées. De préférence, l'empreinte comporte au moins les deux arcs dentaires et au moins une partie du palais du patient. Typiquement l'empreinte comporte l'arc dentaire supérieur 10, l'arc dentaire inférieur 11, le palais 12 ainsi qu'une partie de la mandibule 13 et du maxillaire 14.

L'empreinte numérique peut être réalisée, par exemple, à partir d'une imagerie médicale du patient comme une radiographie en trois dimensions de type Tomodensitométrie Volumique à Faisceau Conique (TVFC plus connu sous l'acronyme anglais de CBCT pour Cone Beam Computed Tomography), ou encore un scanner dentaire en trois dimensions ou un scanner dentaire de type Dentascan. L'empreinte numérique est un fichier numérique connu du domaine de la profession dentaire. A cet effet, pour la mise en œuvre de l'invention, on pourra soit créer ladite empreinte numérique (cette étape étant connue de l'art antérieur, elle ne sera pas décrite plus en détail ici) soit la récupérer directement du praticien ayant examiné le patient.

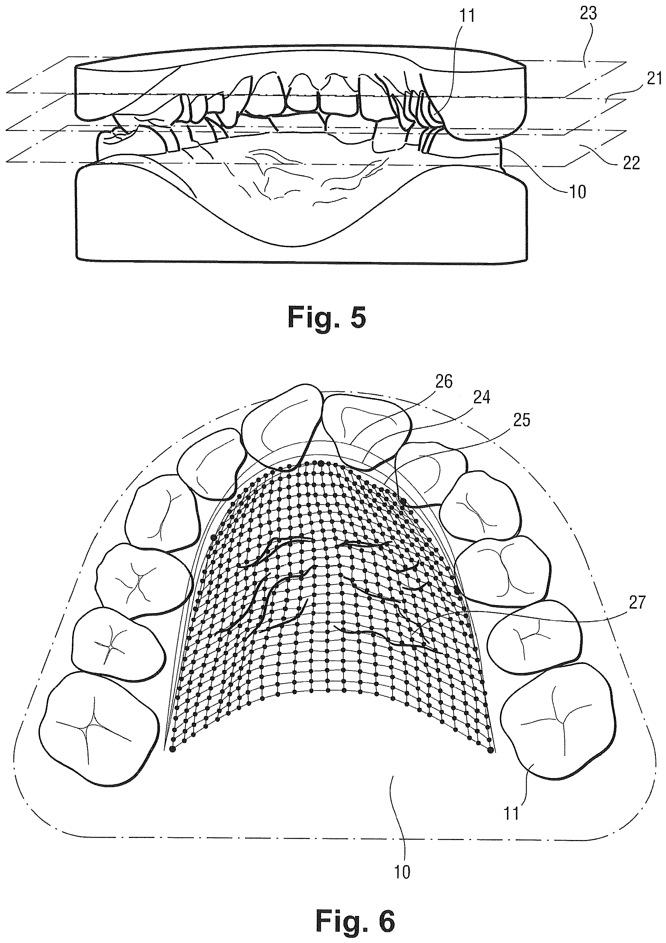

Lors d'une deuxième étape, et comme visible à la figure 5, trois plans distincts sont ensuite créés dans l'empreinte.

Un premier plan 21 est créé au niveau des faces occlusales des deux arcs dentaires 10, 11 afin que ledit premier plan 20 croise le plus de dents possibles. On définit de préférence ledit premier plan 21 afin qu'il croise en priorité et au maximum les dents les plus rentrées dans la cavité buccale (soit les dents les plus encombrantes pour le positionnement de l'appareil 1 dans la cavité buccale) et de manière générale afin qu'il croise le maximum de dents.

Un deuxième plan 22 est créé au niveau de l'arc dentaire supérieur 10. On définit ledit deuxième plan 22 de sorte que, pour un maximum de dents possibles de l'arc dentaire supérieur 10, ledit deuxième plan 22 soit agencé entre les collets (étant rappelé qu'un collet est la partie de la dent entre la couronne et la racine) et le milieu des dents.

Un troisième plan 23 est créé au niveau de l'arc dentaire inférieur 11. On définit ledit troisième plan 23 de sorte que, pour un maximum de dents possibles de l'arc dentaire inférieur 11, ledit troisième plan 23 soit agencé au niveau des collets.

De préférence on définit également les trois plans 21, 22, 23 afin qu'au moins deux des trois plans soient parallèles entre eux. Préférentiellement, on définit les trois plans 21, 22, 23 afin qu'ils soient tous parallèles entre eux.

En référence à la figure 6 (la partie inférieure de la cavité buccale n'étant pas visible ici pour simplifier la lecture de la figure 6), pour chacun des trois plans 21, 22, 23 précités, on définit alors une courbe sensiblement en forme de U pour suivre l'arc ou les arcs dentaires associés au plan considéré sans toutefois que ladite courbe soit au contact des dents. Comme indiqué ci-dessus, on choisit de préférence les courbes pour qu'elles s'étendent entre 1 millimètre et 3 millimètres, et de préférence entre 1.5 et 2.5 millimètres, de la face palatine de l'arc dentaire supérieur 10 et/ou de la face linguale de l'arc dentaire inférieur 11 selon le plan considéré.

De préférence, la courbe 25 du deuxième plan 22 est également définie pour ne pas toucher la papille rétro-incisive afin que l'appareil 1 ne touche pas ladite papille. En effet, pour un bon positionnement de la langue, le bout de la langue doit venir appuyer sur les crêtes palatines soit sur une zone du palais en retrait de la papille rétro-incisive située juste derrière l'arc dentaire supérieur.

De façon particulière, chaque courbe 24, 25, 26 passe par au moins cinq points du plan associé.

Pour le premier plan 21 les cinq points sont :
- un premier point situé à égale distance des quatre incisives centrales,
- un deuxième point situé à égale distance des deux prémolaires et deux canines du côté gauche,
- un troisième point situé à égale distance des deux prémolaires et des deux canines du côté droit,
- un quatrième point situé à égale distance des deux premières molaires et des deux deuxièmes molaires du côté gauche,
- un cinquième point situé à égale distance des deux premières molaires et des deux deuxièmes molaires du côté droit.

Pour le deuxième plan 22 les cinq points sont :
- un premier point situé à égale distance des deux incisives supérieures,
- un deuxième point situé à égale distance de la prémolaire et de la canine supérieures du côté gauche,
- un troisième point situé à égale distance de la prémolaire et de la canine supérieures du côté droit,
- un quatrième point situé à égale distance de la première molaire et de la deuxième molaire supérieures du côté gauche,
- un cinquième point situé à égale distance de la première molaire et de la deuxième molaire supérieures du côté droit.

Pour le troisième plan 23 les cinq points sont :
- un premier point situé à égale distance des deux incisives inférieures,
- un deuxième point situé à égale distance de la prémolaire et de la canine inférieures du côté gauche,
- un troisième point situé à égale distance de la prémolaire et de la canine inférieures du côté droit,
- un quatrième point situé à égale distance de la première molaire et de la deuxième molaire inférieures du côté gauche,
- un cinquième point situé à égale distance de la première molaire et de la deuxième molaire inférieures du côté droit.

En option, on peut alors créer une modélisation en trois dimensions 27 du palais s'étendant jusqu'à la courbe 25 formée dans le deuxième plan 22, par exemple par filtrage. On note que l'on peut aussi ne pas créer une modélisation aussi finalisée 27, la seule définition des courbes 25 étant suffisante pour modéliser au moins une partie du palais du patient.On crée ensuite un contour externe du modèle de l'appareil 1 à partir des trois courbes précitées 24, 25, 26 (et optionnellement de la modélisation 27) formant ainsi respectivement une face externe du support 2 et de la bordure 3. On note en particulier que les trois courbes précitées 24, 25, 26 permettent de personnaliser le support 2 mais également la bordure 3 au patient. La bordure 3 est lisse néanmoins elle suit une courbure particulière propre au patient.

Ceci permet de définir à terme un appareil 1 pouvant tenir dans la cavité buccale par simple adhésion au palais.

En référence à la figure 1 on extrude alors ledit contour externe pour modéliser l'appareil 1.

De la sorte on obtient une modélisation de l'appareil 1 propre au patient et à son anatomie. On s'assure ainsi que l'appareil 1 ne va pas être au contact de l'une des dents du patient mais également que l'appareil 1 va bien adhérer au palais. On s'assure également ici que l'appareil 1 ne viendra pas toucher la papille rétro-incisive.

On fabrique alors l'appareil 1 à partir de ce modèle, par exemple par impression en trois dimensions. On peut ainsi s'aider d'une fabrication assistée par ordinateur.

On fabrique ainsi un appareil 1 adapté à chaque patient de manière relativement simple et peu coûteuse.

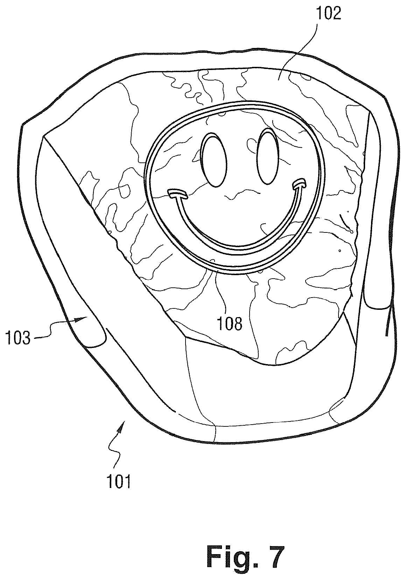

En référence à la figure 7, l'appareil selon le deuxième mode de réalisation de l'invention est identique à l'appareil selon le premier mode de réalisation à la différence que l'appareil 101 comporte au moins une zone d'accueil de la langue.

De façon particulière, cette zone d'accueil est ici une zone en relief 108 agencée sur l'appareil 101 sensiblement au niveau où la pointe de la langue de l'utilisateur doit normalement venir reposer et appuyer lors d'une déglutition.

Il s'avère ainsi relativement simple pour un patient d'apprendre à positionner sa langue correctement puisqu'il lui suffit de sentir la zone en relief 108 avec le bout de sa langue pour savoir que sa langue est correctement placée.

De préférence, cette zone d'accueil est conformée en un dessin (figure, chiffre, lettre, image, émoticône ...). Ceci permet de donner à l'appareil 101 un aspect plus rassurant et/ou plus ludique ce qui facilite l'acceptation du traitement et de l'appareil 101 notamment chez les jeunes enfants.

L'invention n'est pas limitée aux modes de réalisation particuliers qui viennent d'être décrits, mais englobe au contraire toute variante de réalisation entrant dans le cadre de l'invention.

En particulier, bien qu'ici tout le support simule le palais, seule une partie du support pourra être conformée pour simuler le palais. Par exemple la face principale interne du support, que la langue du patient peut toucher, pourra être lisse.

De la même façon bien qu'ici la bordure soit lisse, la bordure pourra être conformée pour simuler la portion du palais qu'elle représente.

Si l'appareil comporte une zone d'accueil de la langue, en variante ou en complément, ladite zone pourra prendre un autre aspect que ce qui a été indiqué. Par exemple la zone d'accueil pourra comporter un orifice ménagé dans l'appareil. Le patient pourra par exemple toucher réellement son palais à travers l'appareil lors de sa rééducation linguale. Bien entendu un même appareil pourra comprendre plusieurs zones d'accueil identiques ou différentes.

L'appareil pourra comporter d'autres éléments que ce qui a été indiqué. Par exemple, l'appareil pourra comporter un capteur de pression. Ledit capteur pourra par exemple, bien que non exclusivement, mesurer la poussée exercée par la langue lors d'une déglutition.

L'appareil pourra également comporter un ou plusieurs capteurs afin qu'il soit possible de réaliser des statistiques à partir des informations indiquées par ces capteurs. Ces capteurs pourront être associés à au moins une mémoire portée par l'appareil pour enregistrer les données fournies par les capteurs et/ou à des moyens de communication à distance avec un organe de calcul traiter les données fournies par les capteurs et/ou un organe de calcul miniature directement porté par l'appareil pour traiter les données au moins en partie au niveau de l'appareil. On pourra ainsi effectuer un suivi propre à chaque patient et de préférence sur le long terme.

Le ou les capteurs pourront être choisis dans la liste suivante : capteur de pression, capteur de position, capteur de distance, capteur de déviation, capteur de déplacement, capteur thermique, capteur biochimique, capteur optique, caméra ...

Bien qu'ici l'appareil orthodontique soit conformé pour s'étendre en retrait des arcs dentaires, l'appareil orthodontique pourra être conformé pour s'étendre jusqu'à au moins l'un des arcs dentaires et/ou au plan d'occlusion. L'appareil orthodontique pourra ainsi toucher les dents.

Par ailleurs, bien qu'ici l'appareil orthodontique s'étende entièrement à l'intérieur de la cavité buccale du patient, l'appareil orthodontique pourra s'étendre en partie à l'extérieur de la cavité buccale. On pourra également envisager que l'appareil soit solidarisé, même de manière temporaire, à l'un ou les deux arcs dentaires du patient. On note toutefois que du fait de sa conformation à la forme du palais, l'appareil pourra de toute façon tenir seul contre le palais sans l'aide de ces arcs dentaires (qui ne seront là que pour par exemple effectuer un autre travail sur les dents). Il est juste envisageable que l'appareil soit associé à un autre dispositif présentant une attache aux arcs dentaires même si l'appareil peut tenir seul contre le palais.

## Revendications

1. Appareil orthodontique pour un patient, comprenant :
- un support (2) dont au moins une face externe est conformée pour s'ajuster à la forme du palais du patient, le support étant destiné à être positionné contre le palais du patient,
- une bordure (3) périphérique dudit support destinée à être agencée en regard d'une face palatine de l'arc dentaire supérieur lorsque le support est posé contre le palais, la bordure comportant une première portion (5) pour s'étendre sensiblement du collet de l'arc dentaire inférieur jusqu'à sensiblement le plan d'occlusion, une deuxième portion (6) pour s'étendre sensiblement du plan d'occlusion jusqu'à sensiblement le collet de l'arc dentaire supérieur et une troisième portion (7) pour s'étendre sensiblement depuis le collet de l'arc dentaire supérieur jusqu'aux bords du palais, la première portion ayant une épaisseur supérieure à celle du reste de l'appareil (1) et l'appareil (1) étant conformé de sorte que lorsque l'appareil (1) est en position dans la cavité buccale, la bordure (3) ne soit pas en contact avec les dents du patient.

2. Appareil selon la revendication 1, conformé pour s'étendre entièrement à l'intérieur de la cavité buccale du patient.

3. Appareil selon la revendication 1 ou la revendication 2, dans lequel la face interne du support (2) est également conformée pour reproduire la forme du palais.

4. Appareil selon la revendication 1 ou la revendication 2 ou la revendication 3, dans lequel la bordure (3) est lisse.

5. Appareil selon l'une des revendications précédentes, dans laquelle la bordure (3) est conformée globalement en U.

6. Appareil selon l'une des revendications précédentes, dans lequel la bordure (3) présente une hauteur supérieure à une hauteur de l'arc dentaire supérieur afin de dépasser du plan d'occlusion.

7. Appareil selon la revendication 6, dans lequel la bordure (3) s'étend au moins sur la hauteur combinée de l'arc dentaire supérieur et de l'arc dentaire inférieur.

8. Appareil selon l'une des revendications précédentes, dans lequel la bordure (3) s'étend entre 1 millimètre et 3 millimètres en retrait d'une face palatine de l'arc dentaire supérieur.

9. Appareil selon l'une des revendications précédentes, comprenant une zone d'accueil de la langue du patient comprenant au moins un orifice.

10. Appareil selon l'une des revendications précédentes, comprenant une zone d'accueil de la langue du patient comprenant une zone en relief (108).

11. Procédé de fabrication d'un appareil orthodontique (1) selon l'une quelconque des revendications précédentes, comprenant les étapes de :
- obtenir une empreinte en trois dimensions d'au moins une partie de la cavité buccale du patient,
- créer dans ladite empreinte au moins un plan au niveau des faces occlusales (21) des deux arcs dentaires de ladite empreinte pour modéliser au moins une partie du palais du patient,
- créer une modélisation en trois dimensions de l'appareil orthodontique,
- créer l'appareil orthodontique (1) à partir de ladite modélisation.

12. Procédé de fabrication selon la revendication 11, dans lequel le plan est créé afin qu'il croise le plus de dents possibles.

13. Procédé de fabrication selon la revendication 11 ou la revendication 12, dans lequel le plan est créé afin qu'il croise en priorité et au maximum les dents les plus rentrées dans la cavité buccale.

14. Procédé de fabrication selon l'une des revendications 11-13, dans lequel on créé dans l'empreinte au moins deux autres plans, l'un associé à l'arc dentaire supérieur et l'autre à l'arc dentaire inférieur pour modéliser au moins une partie du palais du patient.

15. Procédé de fabrication selon l'une des revendications 11-14, dans lequel on définit une courbe passant par au moins cinq points du plan.

16. Procédé de fabrication selon l'une des revendications 11-15, dans lequel on prend également en compte la papille palatine et les bords latéraux de la mandibule pour la modélisation en trois dimensions de l'appareil orthodontique.

## Patentansprüche

1. Kieferorthopädische Vorrichtung für einen Patienten, umfassend:
- einen Träger (2), von dem zumindest eine Außenfläche so geformt ist, dass sie sich an die Form des Gaumens des Patienten anpasst, wobei der Träger dazu bestimmt ist, gegen den Gaumen des Patienten positioniert zu werden,
- einen Umfangsrand des genannten Trägers, der dazu bestimmt ist, gegenüber einer Gaumenfläche des oberen Zahnbogens angeordnet zu werden, wenn der Träger gegen den Gaumen gelegt wird, wobei der Rand einen ersten Abschnitt (5) umfasst, um sich ungefähr vom Hals des unteren Zahnbogens bis ungefähr zur Okklusionsebene zu erstrecken, einen zweiten Abschnitt (6), um sich ungefähr von der Okklusionsebene bis ungefähr zum Hals des oberen Zahnbogens zu erstrecken, und einen dritten Abschnitt (7), um sich ungefähr von dem Hals des oberen Zahnbogens bis zu den Rändern des Gaumens zu erstrecken, wobei der erste Abschnitt eine Dicke hat, die größer als die des Rests der Vorrichtung (1) ist, und wobei die Vorrichtung (1) so geformt ist, dass, wenn die Vorrichtung (1) in Position in der Mundhöhle ist, der Rand (3) nicht mit den Zähnen des Patienten in Kontakt ist.

2. Vorrichtung nach Anspruch 1, die so geformt ist, dass sie sich vollständig im Inneren der Mundhöhle des Patienten erstreckt.

3. Vorrichtung nach Anspruch 1 oder Anspruch 2, bei der die Innenfläche des Trägers (2) ferner so geformt ist, dass sie die Form des Gaumens nachbildet.

4. Vorrichtung nach Anspruch 1 oder Anspruch 2 oder Anspruch 3, bei der der Rand (3) glatt ist.

5. Vorrichtung nach einem der vorhergehenden Ansprüche, bei der der Rand (3) im Ganzen die Form eines U hat.

6. Vorrichtung nach einem der vorhergehenden Ansprüche, bei der der Rand (3) eine Höhe aufweist, die höher als eine Höhe des oberen Zahnbogens ist, um über die Okklusionsebene hinauszugehen.

7. Vorrichtung nach Anspruch 6, bei der sich der Rand (3) mindestens über die kombiniete Höhe des oberen Zahnbogens und des unteren Zahnbogens erstreckt.

8. Vorrichtung nach einem der vorhergehenden Ansprüche, bei der der Rand (3) zwischen 1 Millimeter und 3 Millimetern von einer Gaumenfläche des oberen Zahnbogens zurückversetzt ist.

9. Vorrichtung nach einem der vorhergehenden Ansprüche, umfassend eine Aufnahmezone zur Aufnahme der Zunge des Patienten, wobei die Aufnahmezone mindestens eine Öffnung umfasst.

10. Vorrichtung nach einem der vorhergehenden Ansprüche, umfassend eine Aufnahmezone zur Aufnahme der Zunge des Patienten, wobei die Aufnahmezone eine Reliefzone (108) umfasst.

11. Verfahren zur Herstellung einer kieferorthopädischen Vorrichtung (1) nach einem der vorhergehenden Ansprüche, umfassend die Schritte:
- Erhalten eines dreidimensionalen Abdrucks mindestens eines Teils der Mundhöhle des Patienten,
- Erzeugen mindestens einer Ebene in dem genannten Abdruck im Bereich der okklusalen Flächen (21) der beiden Zahnbögen des genannten Abdrucks, um mindestens einen Teil des Gaumens des Patienten zu modellieren,
- Erzeugen einer dreidimensionalen Modellierung der kieferorthopädischen Vorrichtung,
- Erzeugen der kieferorthopädischen Vorrichtung (1) anhand der genannten Modellierung.

12. Verfahren zur Herstellung nach Anspruch 11, bei dem die Ebene erzeugt wird, damit sie so viele Zähne wie möglich kreuzt.

13. Verfahren zur Herstellung nach Anspruch 11 oder Anspruch 12, bei dem die Ebene erzeugt wird, damit sie vorrangig und maximal die Zähne kreuzt, die am weitesten in die Mundhöhle zurückversetzt sind.

14. Verfahren zur Herstellung nach einem der Ansprüche 11-13, bei dem man in dem Abdruck mindestens zwei weitere Ebenen erzeugt, von denen eine mit dem oberen Zahnbogen verknüpft ist und die andere mit dem unteren Zahnbogen verknüpft ist, um mindestens einen Teil des Gaumens des Patienten zu modellieren.

15. Verfahren zur Herstellung nach einem der Ansprüche 11-14, bei dem man eine Kurve definiert, die durch mindestens fünf Punkte der Ebene geht.

16. Verfahren zur Herstellung nach einem der Ansprüche 11-15, bei dem man ebenso die Gaumenpapille und die Seitenränder des Unterkiefers für die dreidimensionale Modellierung der kieferorthopädischen Vorrichtung berücksichtigt.

## Claims

1. An orthodontic appliance for a patient comprising:
- a support (2) of which at least one outer face is shaped to conform to the shape of the patient's palate, the support being intended to be positioned against the patient's palate,
- a peripheral rim (3) of said support intended to be arranged at least facing a palatal face of the upper dental arch when the support is placed against the palate, the rim including a first portion (5) that extends substantially from the collar of the lower dental arch to substantially the occlusion plane, a second portion (6) for extending substantially from the occlusion plane to substantially the collar of the upper dental arch and a third portion (7) that extends substantially from the collar of the upper dental arch to the edges of the palate, the first portion having a greater thickness than the rest of the appliance (1) and the appliance (1) being designed so that when the appliance (1) is in position in the buccal cavity, the rim (3) is not in contact with the patient's teeth.

2. The appliance according to claim 1, shaped to extend fully into the patient's buccal cavity.

3. An appliance according to claim 1 or claim 2, wherein the inner surface of the support (2) is also shaped to reproduce the shape of the palate.

4. An appliance according to claim 1 or claim 2 or claim 3, wherein the rim (3) is smooth.

5. An appliance according to one of the preceding claims, wherein the rim (3) globally has the shape of a U.

6. An appliance according to one of the preceding claims, wherein the rim (3) has a height greater than a height of the upper dental arch in order to extend beyond the occlusion plane.

7. An appliance according to claim 6, wherein the rim (3) extends at least over the combined height of the upper and lower dental arch.

8. An appliance according to one of the preceding claims, wherein the rim (3) extends between 1mm and 3mm back from a palatal face of the upper dental arch.

9. An appliance according to one of the preceding claims, comprising a patient's tongue reception area comprising at least one orifice.

10. An appliance according to one of the preceding claims, comprising a patient's tongue reception area comprising a raised area (108).

11. A method for manufacturing an orthodontic appliance (1) according to one of the preceding claims, comprising the following steps:
- obtaining a three-dimensional impression of at least part of the patient's buccal cavity,
- creating in said impression at least one plane at the occlusal surfaces (21) of the two dental arches of said impression to model at least a part of the patient's palate,
- creating a three-dimensional model of the orthodontic appliance,
- creating the orthodontic appliance (1) from said model.

12. A manufacturing method according to claim 11, wherein the plane is created so that it crosses as many teeth as possible.

13. A manufacturing method according to claim 11 or claim 12, wherein the plane is created so that it crosses first and foremost and at most the most in-turned teeth in the buccal cavity.

14. A manufacturing method according to one of claims 11-13, wherein at least two other planes are created in the impression, one associated with the upper and the other with the lower dental arch to model at least a part of the patient's palate.

15. A manufacturing method according to one of the claims 11-14, wherein a curve is defined passing through at least five points of the plane.

16. A manufacturing method according to one of the claims 11-15, wherein the palatal papilla and the lateral rims of the mandible are also taken into account for the three-dimensional modelling of the orthodontic appliance.
